# EUROPEAN PATENT APPLICATION

(11) **EP 3 725 890 A1**
(43) Date of publication of application: **21.10.2020**
(21) Application number: 18889908.2
(22) Date of filing: 12.12.2018
(51) Int. Cl.: C12P 7/64, C12P 1/00, C12P 9/00, C12N 9/16

(54) **METHOD FOR PREPARING SODIUM CYCLIC PHOSPHATIDIC ACID**

(30) Priority: 12.12.2017 JP 2017237364
(71) Applicant: Sansho Co., Ltd., Tokyo 103-0027 (JP)
(72) Inventor: IMAMURA Shigeyuki, Izunokuni-shi, Shizuoka 410-2321 (JP); NOGATA Yoshihiko, Izunokuni-shi, Shizuoka 410-2123 (JP); FUJIWARA Tatsuro, Izunokuni-shi, Shizuoka 410-2143 (JP); MOROHOSHI Toshiro, Isehara-shi, Kanagawa 259-1133 (JP)
(74) Representative: Godemeyer Blum Lenze Patentanwälte Partnerschaft mbB - werkpatent
(86) International application number: PCT/JP2018/045625
(87) International publication number: WO 2019/117187

(57) **Abstract**

It is an object of the present invention to provide a method for easily producing sodium cyclic phosphatidic acid that can be used as food and drink. According to the present invention, provided is a method for producing sodium cyclic phosphatidic acid, comprising a step of allowing a lyso-type phospholipid to react with phospholipase D in the presence of sodium salts in an aqueous medium, and a step of recovering a precipitate or an upper layer liquid that is generated as a result of addition of ethyl alcohol to the obtained reaction solution.

## Description

### Technical Field

The present invention relates to a method for producing sodium cyclic phosphatidic acid.

### Background Art

Cyclic phosphatidic acid (hereinafter occasionally abbreviated as "cPA") has been known to have physiological activity such as inhibition of metastasis and invasion of cancer cells (Non-Patent Document 1), and such cPA is expected to have intended uses as pharmaceutical products including antitumor agents, foods with functional claims, and food products. In addition, since cyclic phosphatidic acid has an action to promote the synthesis of hyaluronic acid *in vivo,* it has been used in cosmetic products.

Conventionally, as methods for producing such cyclic phosphatidic acid, methods for chemically synthesizing cyclic phosphatidic acid (Patent Documents 1 and 2) and methods of utilizing enzymatic reactions, in which phospholipase D is allowed to act on a lyso-type phospholipid (Patent Documents 3 and 4), have been known. Since such cyclic phosphatidic acid is a lipid that is insoluble in water, it is necessary to convert the cyclic phosphatidic acid to water-soluble salts such as sodium salts. Thus, sodium cyclic phosphatidic acid has been prepared according to a method comprising treating chemically synthesized cyclic phosphatidic acid with a strong base such as sodium chloride or sodium hydroxide so as to convert it to sodium salts.

Moreover, there has also been known a method for producing sodium cyclic phosphatidic acid, comprising adding sodium salts to a reaction product obtained by allowing phospholipase D to act on a lyso-type phospholipid (excluding a hydrogenation product) in a system containing an organic solvent and/or water, and then removing the solvent from the reaction product (Patent Document 5).

Furthermore, there has also been known a method for producing 1-acyl-2,3-cyclic phosphatidic acid or a salt thereof, which is characterized in that the method comprises: a step of subjecting lysophosphatidylcholine and actinomyces (genus Actinomadura)-derived phospholipase D to an enzymatic reaction in an aqueous solution, in which the content of sodium atoms is 0.2% by mass or less and the content of calcium atoms is 0.02% by mass or less, at 50°C to 65°C for 4 to 10 hours; a step of adding ethanol to the reaction product, then leaving the mixture at rest at 0°C to 8°C for 15 to 30 hours for conservation, and then filtrating the reaction solution, wherein this step is performed as a post-treatment of the enzymatic reaction; and a step of treating the filtrate obtained as a result of the filtration with an Na-type strongly acidic cation ion exchange resin (Patent Document 6).

### Prior Art Documents

### Patent Documents

Patent Document 1: Japanese Patent Publication (Kokai) No. 6-228169 A (1994)
Patent Document 2: Japanese Patent Publication (Kokai) No. 7-258278 A (1995)
Patent Document 3: Japanese Patent Publication (Kokai) No. 2001-178489 A
Patent Document 4: Japanese Patent Publication (Kokai) No. 2008-222643 A
Patent Document 5: Japanese Patent No. 5933338
Patent Document 6: Japanese Patent Publication (Kokai) No. 2014-93953 A

### Non-Patent Documents

Non-Patent Document 1: Biochemica et Biophysica Acta 15288 (2002), pp.1-7

### Summary of Invention

### Object to be Solved by the Invention

With regard to the method described in the Examples of Patent Document 5, after sodium cyclic phosphatidic acid has been gathered to a chloroform layer, the chloroform is distilled away to obtain the sodium cyclic phosphatidic acid. However, taking into consideration the intended use of sodium cyclic phosphatidic acid as a food product, it is desired to apply a production method that does not use chloroform. In addition, since the method disclosed in Patent Document 6 comprises a step of treating the filtrate with a Na-type strongly acidic cation ion exchange resin, the operations have been complicated.

It is an object of the present invention to provide a method for easily producing sodium cyclic phosphatidic acid that can be used as food and drink.

### Means for Solving the Object

As a result of intensive studies conducted regarding a method for producing sodium cyclic phosphatidic acid, the present inventors have discovered that sodium cyclic phosphatidic acid can be easily obtained by allowing a lyso-type phospholipid to react with phospholipase D in the presence of sodium salts in an aqueous medium, and then recovering a precipitate or an upper layer liquid that is generated as a result of addition of ethyl alcohol to the obtained reaction solution. The present invention has been completed based on the aforementioned findings.

Specifically, the present invention provides the following inventions.
(1) A method for producing sodium cyclic phosphatidic acid, comprising a step of allowing a lyso-type phospholipid to react with phospholipase D in the presence of sodium salts in an aqueous medium, and a step of recovering a precipitate or an upper layer liquid that is generated as a result of addition of ethyl alcohol to the obtained reaction solution.
(2) The method for producing sodium cyclic phosphatidic acid according to (1), wherein the lyso-type phospholipid is a soybean-derived lyso-type phospholipid.
(3) The method for producing sodium cyclic phosphatidic acid according to (1) or (2), wherein the step of allowing a lyso-type phospholipid to react with phospholipase D in the presence of sodium salts in an aqueous medium is a step of allowing a lyso-type phospholipid to react with phospholipase D in a sodium acetate-acetic acid buffer solution.
(4) The method for producing sodium cyclic phosphatidic acid according to any one of (1) to (3), wherein the step of allowing a lyso-type phospholipid to react with phospholipase D in the presence of sodium salts in an aqueous medium is a step of allowing a lyso-type phospholipid to react with phospholipase D in a sodium acetate-acetic acid buffer solution containing a chelating agent.
(5) The method for producing sodium cyclic phosphatidic acid according to (4), wherein the chelating agent is EDTA.
(6) The method for producing sodium cyclic phosphatidic acid according to any one of (3) to (5), wherein a precipitate generated as a result of addition of ethyl alcohol to the reaction solution is recovered.
(7) The method for producing sodium cyclic phosphatidic acid according to (1) or (2), wherein the step of allowing a lyso-type phospholipid to react with phospholipase D in the presence of sodium salts in an aqueous medium is a step of allowing a lyso-type phospholipid to react with phospholipase D in a sodium citrate-citric acid buffer solution.
(8) The method for producing sodium cyclic phosphatidic acid according to (7), wherein an upper layer liquid generated as a result of addition of ethyl alcohol to the reaction solution is recovered.

### Advantageous Effects of Invention

According to the present invention, sodium cyclic phosphatidic acid that can be used as food and drink can be easily produced.

### Brief Description of Drawings

Fig. 1 shows the results obtained by detecting sodium cyclic lysophosphatidic acid according to thin layer chromatography.
Fig. 2 shows the results obtained by detecting sodium cyclic lysophosphatidic acid according to thin layer chromatography.

### Embodiment of Carrying out the Invention

Hereinafter, the present invention will be described in more detail.

The method for producing sodium cyclic phosphatidic acid according to the present invention is characterized in that it comprises a step of allowing a lyso-type phospholipid to react with phospholipase D in the presence of sodium salts in an aqueous medium, and a step of recovering a precipitate or an upper layer liquid (a supernatant) that is generated as a result of addition of ethyl alcohol to the obtained reaction solution. The method of the present invention can be carried out without using organic solvents (i.e., organic solvents other than ethyl alcohol), such as chloroform, methylene chloride, toluene, ethyl ether, ethyl acetate, or hexane.

Examples of a known lyso-type phospholipid may include those having different fatty acid species and molecular species having an ether or vinyl ether bond. These lyso-type phospholipids are available as commercially available products.

As such a lyso-type phospholipid, a soybean-derived lyso-type phospholipid, a yolk-derived lyso-type phospholipid, a corn-derived lyso-type phospholipid, or the like can be used. Among these, a soybean-derived lyso-type phospholipid is preferably used.

As such a lyso-type phospholipid, for example, partially hydrolyzed lysolecithin, which is prepared by treating lecithin used as a raw material, fractionated lecithin, etc. with phospholipase A₂, can be used. For example, soybean lysolecithin can be used. As such soybean lysolecithin, a commercially available product can be purchased.

In the present invention, a lyso-type phospholipid is allowed to react with phospholipase D in the presence of sodium salts in an aqueous medium. For example, a lyso-type phospholipid is allowed to react with phospholipase D in a sodium acetate-acetic acid buffer solution or in a sodium citrate-citric acid buffer solution. By allowing sodium salts to exist in the reaction system, sodium cyclic phosphatidic acid can be produced.

The reaction of a lyso-type phospholipid with phospholipase D may be carried out in the presence of a chelating agent. Examples of the chelating agent used in the present invention may include sodium ethylenediaminetetraacetate (EDTA), diethylenetriamine pentaacetic acid, glycol ether diamine tetraacetic acid, citric acid, tartaric acid, and phytic acid. Among these, sodium ethylenediaminetetraacetate (EDTA), diethylenetriamine pentaacetic acid, and glycol ether diamine tetraacetic acid are preferable, and the most preferred chelating agent is EDTA.

In the reaction of a lyso-type phospholipid with phospholipase D, calcium ions are preferably not present. If calcium ions are present, such calcium ions are preferably present in a trace amount.

The phospholipase D used in the present invention is not particularly limited, as long as it generates cPA when it is allowed to act on a lyso-type phospholipid. The phospholipase D derived from Streptomyces sp. or Actinomadula sp. is particularly preferably used.

The reaction of a lyso-type phospholipid with phospholipase D can be carried out, for example, by increasing the temperature to a range of 25°C to 50°C, preferably to a range of 30°C to 45°C, and then allowing the lyso-type phospholipid to react with the phospholipase D for approximately 5 to 30 hours, while continuously stirring.

More specifically, the reaction of a lyso-type phospholipid with phospholipase D can be carried out by the following procedures.
(Method 1) A lyso-type phospholipid (soybean lysolecithin, etc.) is added to a sodium acetate-acetic acid buffer solution or a sodium citrate-citric acid buffer solution (pH 4.0 to 7.0, preferably pH 5.0 to 6.0), and it is then dispersed and dissolved therein. Thereafter, phospholipase D is dispersed in a small amount of purified water, as desired, and it is then added to the above-obtained solution. The obtained mixed solution is stirred at 25°C to 50°C for 5 to 30 hours.
(Method 2) A lyso-type phospholipid (soybean lysolecithin, etc.) is added to a sodium acetate-acetic acid buffer solution containing EDTA (pH 4.0 to 7.0, preferably pH 5.0 to 6.0), and it is then dispersed and dissolved therein. Thereafter, phospholipase D is dispersed in a small amount of purified water, as desired, and it is then added to the above-obtained solution. The obtained mixed solution is stirred at 25°C to 50°C for 5 to 30 hours.

In the present invention, a lyso-type phospholipid is allowed to react with phospholipase D in the presence of sodium salts in an aqueous medium, and thereafter, a precipitate or an upper layer liquid generated as a result of addition of ethyl alcohol to the obtained reaction solution is recovered. The present inventors have found that, in the present invention, sodium cyclic lysophosphatidic acid generated as a result of the reaction of a lyso-type phospholipid with phospholipase D in the presence of a sodium acetate-acetic acid buffer solution in an aqueous medium is precipitated by addition of ethyl alcohol, and the inventors have succeeded in easily recovering sodium cyclic lysophosphatidic acid (i.e., without using an organic solvent, and without performing a treatment with a strongly acidic cation exchange resin, etc.) by recovering the obtained precipitate. Moreover, the present inventors have found that, in the present invention, sodium cyclic lysophosphatidic acid generated as a result of the reaction of a lyso-type phospholipid with phospholipase D in the presence of a sodium citrate-citric acid buffer solution in an aqueous medium is present in an upper layer liquid from the upper layer liquid and a lower layer liquid, which have been generated by adding ethyl alcohol to the reaction solution, stirring the mixture, and then leaving it at rest. Then, the inventors have succeeded in easily recovering sodium cyclic lysophosphatidic acid (i.e., without using an organic solvent, and without performing a treatment with a strongly acidic cation exchange resin, etc.) by recovering the aforementioned upper layer liquid.

Besides, the recovery of a precipitate by addition of ethyl alcohol may also be carried out multiple times.

In one embodiment of the present invention, a precipitate generated as a result of addition of ethyl alcohol (first addition) is recovered by a centrifugal operation (e.g. 3000 rotations, 5 minutes), and the recovered precipitate is then dissolved in purified water. In one embodiment of the present invention, a precipitate generated as a result of addition of ethyl alcohol (second addition) to the obtained solution may be recovered by a centrifugal operation (e.g. 3000 rotations, 5 minutes).

In addition, in another embodiment of the present invention, ethyl alcohol is added to the reaction solution and the obtained mixture is then stirred, followed by leaving the reaction mixture at rest. Thereafter, a lower layer portion is removed by liquid separation, so that an upper layer liquid can be obtained. This upper layer liquid is recovered and is then concentrated under reduced pressure to remove ethanol. Thereafter, the residue is dissolved in purified water, followed by freeze-drying, so that powders containing sodium cyclic lysophosphatidic acid can be obtained.

As desired, the above recovered sodium cyclic lysophosphatidic acid is dissolved in purified water, followed by freeze-drying, so that powders containing sodium cyclic lysophosphatidic acid can be obtained.

The present invention will be described in the following examples. However, these examples are not intended to limit the scope of the present invention. It is to be noted that the soybean lysolecithin (SLP-White Lyso) used in the following examples contains a small amount of calcium.

### Examples

### Example 1

10 g of Soybean lysolecithin (LPC70) was added to 100 ml of a 1 M sodium acetate-acetic acid buffer solution (pH 5.5), and it was dispersed and dissolved therein. Thereafter, 400 mg of phospholipase D (manufactured by Meito Sangyo Co., Ltd.; derived from Actinomadura) was dispersed in a small amount of purified water, and the obtained solution was then added to the above-obtained solution. The obtained mixture was stirred at 40°C for 16 hours. After completion of the reaction, a precipitate generated as a result of addition of 100 ml of ethyl alcohol to the reaction mixture was recovered by performing centrifugation at 3000 rotations for 5 minutes, and was then dissolved in 50 ml of purified water. After that, a precipitate generated as a result of addition of 50 ml of ethyl alcohol to the obtained solution was recovered by performing centrifugation at 3000 rotations for 5 minutes, and was then dissolved in 30 ml of purified water. The thus obtained solution was freeze-dried to obtain 4.2 g of powders containing sodium cyclic lysophosphatidic acid.

### Example 2

10 g of Soybean lysolecithin (LPC70) was added to 100 ml of a 1 M sodium acetate-acetic acid buffer solution (pH 5.5) containing 10 mM EDTA, and it was dispersed and dissolved therein. Thereafter, 400 mg of phospholipase D (manufactured by Meito Sangyo Co., Ltd.; derived from Actinomadura) was dispersed in a small amount of purified water, and the obtained solution was then added to the above-obtained solution. The obtained mixture was stirred at 40°C for 16 hours. After completion of the reaction, a precipitate generated as a result of addition of 100 ml of ethyl alcohol to the reaction mixture was recovered by performing centrifugation at 3000 rotations for 5 minutes, and was then dissolved in 50 ml of purified water. After that, a precipitate generated as a result of addition of 50 ml of ethyl alcohol to the obtained solution was recovered by performing centrifugation at 3000 rotations for 5 minutes, and was then dissolved in 35 ml of purified water. The thus obtained solution was freeze-dried to obtain 4.0 g of powders.

### Example 3

10 g of White Lyso (manufactured by Tsuji Oil Mills co., Ltd.) was dissolved in 100 ml of a 1 M sodium citrate-citric acid buffer solution (pH 6.0), and 200 mg of Actinomadura-derived phospholipase D (manufactured by Meito Sangyo Co., Ltd.) was then added thereto, so that an enzymatic reaction was carried out at 40°C for 16 hours. Thereafter, 100 ml of 99% ethanol was added to the reaction solution, and the obtained mixture was then stirred. After that, the reaction mixture was left at rest at room temperature for 1 hour, and a lower layer portion was then removed by liquid separation to obtain 135 ml of an upper layer liquid. This upper layer liquid was recovered and was then concentrated under reduced pressure, using a rotary evaporator, to remove the ethanol. Thereafter, the residue was dissolved in 40 ml of purified water, followed by freeze-drying, to obtain 5.2 g of powders containing sodium cyclic lysophosphatidic acid.

### Example 4

10 g of Soybean lysolecithin (LPC70), manufactured by Tsuji Oil Mills co., Ltd., was dissolved in 100 ml of a 1 M sodium citrate-citric acid buffer solution (pH 6.0), and 200 mg of Actinomadura-derived phospholipase D (manufactured by Meito Sangyo Co., Ltd.) was then added thereto, so that an enzymatic reaction was carried out at 40°C for 16 hours. Thereafter, 100 ml of 99% ethanol was added to the reaction solution, and the obtained mixture was then stirred. After that, the reaction mixture was left at rest at room temperature for 1 hour, and a lower layer portion was then removed by liquid separation to obtain 135 ml of an upper layer liquid. This upper layer liquid was recovered and was then concentrated under reduced pressure, using a rotary evaporator, to remove the ethanol. The residue was dissolved in 40 ml of purified water, followed by freeze-drying, to obtain 4.5 g of powders containing sodium cyclic lysophosphatidic acid.

### < Detection of sodium cyclic lysophosphatidic acid according to thin layer chromatography >

The powders containing sodium cyclic lysophosphatidic acid (10 mg) obtained in each of Example 1, Example 3 and Example 4 were weighed, and were then dissolved in 1 ml of chloroform : methanol : water (60 : 30 : 5, V/V). After that, 5 µl of the obtained solution was spotted on a thin layer plate manufactured by Merck, and was then developed thereon using a mixed solvent of chloroform : methanol : acetic acid: water (60 : 30 : 3 : 5, V/V). Thereafter, the plate was dried, and an 8% phosphoric acid-2% sulfuric acid solution containing 3% copper acetate was then sprayed onto the plate. After that, the plate was heated at 150°C for 3 minutes, and spots of sodium cyclic lysophosphatidic acid were then confirmed. Sodium cyclic lysophosphatidic acid prepared by the method described in Japanese Patent No. 593338 was used as a control.

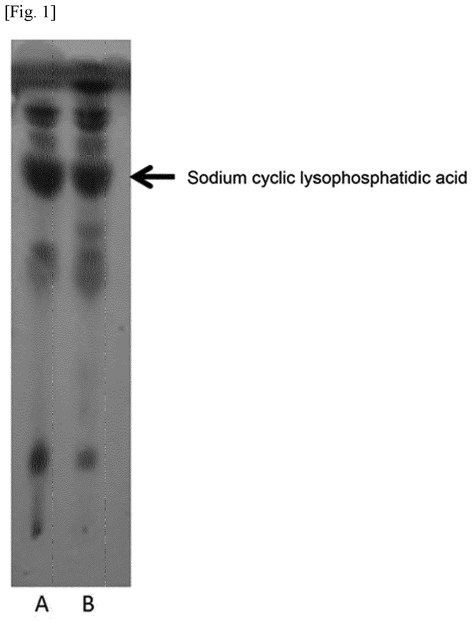

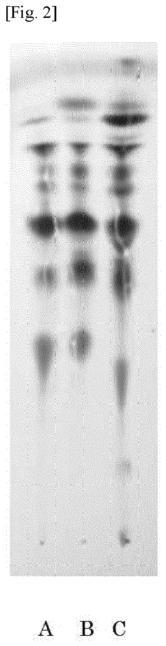

The results are shown in Fig. 1 and Fig. 2.

Fig. 1A shows the results of the sodium cyclic lysophosphatidic acid prepared by the method described in Japanese Patent No. 593338, and Fig. 1B shows the results of a sample prepared in Example 1.

As shown in Fig. 1, the sodium cyclic lysophosphatidic acid obtained in Example 1 had an Rf value that was identical to that of the sodium cyclic lysophosphatidic acid prepared by the method described in Japanese Patent No. 593338 and used as a control.

Fig. 2A shows the results of the sodium cyclic lysophosphatidic acid prepared by the method described in Japanese Patent No. 593338, and Fig. 2B shows the results of a sample prepared by the method described in Example 4. In addition, Fig. 2C shows the results of a sample prepared by the method described in Example 3. As shown in Fig. 2, the sodium cyclic lysophosphatidic acid obtained in each of Examples 3 and 4 had an Rf value that was identical to that of the sodium cyclic lysophosphatidic acid prepared by the method described in Japanese Patent No. 593338 and used as a control.

## Claims

1. A method for producing sodium cyclic phosphatidic acid, comprising a step of allowing a lyso-type phospholipid to react with phospholipase D in the presence of sodium salts in an aqueous medium, and a step of recovering a precipitate or an upper layer liquid that is generated as a result of addition of ethyl alcohol to the obtained reaction solution.

2. The method for producing sodium cyclic phosphatidic acid according to claim 1, wherein the lyso-type phospholipid is a soybean-derived lyso-type phospholipid.

3. The method for producing sodium cyclic phosphatidic acid according to claim 1 or 2, wherein the step of allowing a lyso-type phospholipid to react with phospholipase D in the presence of sodium salts in an aqueous medium is a step of allowing a lyso-type phospholipid to react with phospholipase D in a sodium acetate-acetic acid buffer solution.

4. The method for producing sodium cyclic phosphatidic acid according to any one of claims 1 to 3, wherein the step of allowing a lyso-type phospholipid to react with phospholipase D in the presence of sodium salts in an aqueous medium is a step of allowing a lyso-type phospholipid to react with phospholipase D in a sodium acetate-acetic acid buffer solution containing a chelating agent.

5. The method for producing sodium cyclic phosphatidic acid according to claim 4, wherein the chelating agent is EDTA.

6. The method for producing sodium cyclic phosphatidic acid according to any one of claims 3 to 5, wherein a precipitate generated as a result of addition of ethyl alcohol to the reaction solution is recovered.

7. The method for producing sodium cyclic phosphatidic acid according to claim 1 or 2, wherein the step of allowing a lyso-type phospholipid to react with phospholipase D in the presence of sodium salts in an aqueous medium is a step of allowing a lyso-type phospholipid to react with phospholipase D in a sodium citrate-citric acid buffer solution.

8. The method for producing sodium cyclic phosphatidic acid according to claim 7, wherein an upper layer liquid generated as a result of addition of ethyl alcohol to the reaction solution is recovered.
